# EUROPEAN PATENT SPECIFICATION

(11) **EP 1 450 852 B1**
(45) Date of publication and mention of the grant of the patent: **17.01.2007**
(21) Application number: 02804223.2
(22) Date of filing: 04.12.2002
(51) Int. Cl.: A61K 38/45, A61P 3/08

(54) **PTP10D NUCLEIC ACIDS AND PEPTIDES INVOLVED IN THE REGULATION OF ENERGY HOMEOSTASIS**
PTP10D NUKLEINSÄUREN UND PEPTIDE IN DER REGULATION VON ENERGIE-HOMEOSTASE
ACIDES NUCLEIQUES ET PEPTIDES DE PTP10D INTERVENANT DANS LA REGULATION DE L'HOMEOSTASIE ENERGETIQUE

(30) Priority: 04.12.2001 EP 01128844; 07.12.2001 EP 01129138; 02.01.2002 EP 02000010
(43) Date of publication of application: 01.09.2004
(73) Proprietor: DeveloGen Aktiengesellschaft, 37079 Göttingen (DE)
(72) Inventor: MEISE, Martin, 58675 Hemer (DE); EULENBERG, Karsten, 37120 Bovenden (DE); FRITSCH, Rüdiger, 37073 Göttinger (DE); HÄDER, Thomas, 37073 Göttingen (DE); BRÖNNER, Günter, 37077 Göttingen (DE); STEUERNAGEL, Arnd, 37085 Göttingen (DE)
(74) Representative: Weiss, Wolfgang
(86) International application number: PCT/EP2002/013744
(87) International publication number: WO 2003/047611

(56) References cited:
- EP-A- 1 046 715
- WO-A-00/06087
- WO-A-01/57190
- WO-A-01/66706
- DATABASE GENESEQ [Online] EBI; 6 November 2001 (2001-11-06), "HUMAN POLYNUCLEOTIDE SEQ ID NO 499" XP002247110 Database accession no. AAK51954 & WO 01 57190 A (HYSEQ INC.) 9 August 2001 (2001-08-09)
- PATENT ABSTRACTS OF JAPAN vol. 1995, no. 02, 31 March 1995 (1995-03-31) & JP 06 315382 A (TOSHIO SUDA;OTHERS: 01), 15 November 1994 (1994-11-15)

## Description

This invention relates to the use of nucleic acid sequences encoding PTP10D or homologous proteins, and the polypeptides encoded thereby and to the use thereof and to the use of effectors thereof in the treatment of obesity as well as related disorders such as eating disorder, cachexia, diabetes mellitus, hypertension, coronary heart disease, hypercholesterolemia, dyslipidemia.

There are several metabolic diseases of human and animal metabolism, eg., obesity and severe weight loss, that relate to energy imbalance where caloric intake versus energy expenditure is imbalanced. Obesity is one of the most prevalent metabolic disorders in the world. It is a still poorly understood human disease that becomes more and more relevant for western society. Obesity is defined as a body weight more than 20% in excess of the ideal body weight, frequently resulting in a significant impairment of health. Further parameters for defining obesity are waist circumferences, skinfold thickness and bioimpedance (see, inter alia, Kopelman (1999), loc. cit.). Obesity is associated with an increased risk for cardiovascular disease, hypertension, diabetes, hyperlipidaemia and an increased mortality rate. Besides severe risks of illness, individuals suffering from obesity are often isolated socially.

Human obesity is strongly influenced by environmental and genetic factors, whereby the environmental influence is often a hurdle for the identification of (human) obesity genes. Obesity is influenced by genetic, metabolic, biochemical, psychological, and behavioral factors. As such, it is a complex disorder that must be addressed on several fronts to achieve lasting positive clinical outcome. Obese individuals are prone to ailments including: diabetes mellitus, hypertension, coronary heart disease, hypercholesterolemia, dyslipidemia, osteoarthritis, gallstones, cancers of the reproductive organs, and sleep apnea.

Obesity is not to be considered as a single disorder but a heterogeneous group of conditions with (potential) multiple causes. Obesity is also characterized by elevated fasting plasma insulin and an exaggerated insulin response to oral glucose intake (Koltermann, J. Clin. Invest 65, 1980, 1272-1284) and a clear involvement of obesity in type 2 diabetes mellitus can be confirmed (Kopelman, Nature 404, 2000, 635-643).

Hyperlipidemia and elevation of free fatty acids correlate clearly with the "Metabolic Syndrome", which is defined as the linkage between several diseases, including obesity an insulin resistance. This often occurs in the same patients and are major risk factors for development of Type 2 diabetes and cardiovascular disease. It was suggested that the control of lipid levels and glucose levels is required to treat Type 2 Diabetes, heart disease, and other occurances of Metabolic Syndrome (see, for example, Santomauro A. T. et al., (1999) Diabetes, 48(9):1836-1841).

Even if several candidate genes have been described which are supposed to influence the homeostatic system(s) that regulate body mass/weight, like leptin, VCPI, VCPL, or the peroxisome proliferator-activated receptor-gamma co-activator, the distinct molecular mechanisms and/or molecules influencing obesity or body weight/body mass regulations are not known.

Therefore, the technical problem underlying the present invention was to provide for means and methods for modulating (pathological) metabolic conditions influencing body-weight regulation and/or energy homeostatic circuits. The solution to said technical problem is achieved by providing the embodiments characterized in the claims.

Accordingly, the present invention relates to genes with novel functions in body-weight regulation, energy homeostasis, metabolism, and obesity. The present invention discloses specific genes involved in the regulation of body-weight, energy homeostasis, metabolism, and obesity, and thus in disorders related thereto such as eating disorder, cachexia, diabetes mellitus, hypertension, coronary heart disease, hypercholesterolemia, dyslipidemia, osteoarthritis, gallstones, cancer, e.g. cancers of the reproductive organs, and sleep apnea. More particularly, the present invention describes the human PTP10D gene as being involved in those conditions mentioned above.

The term "polynucleotide comprising the nucleotide sequence as shown in GenBank Accession number" relates to the expressible gene of the nucleotide sequences deposited under the corresponding GenBank Accession number. The term "GenBank Accession Number" relates to NCBI GenBank database entries (Ref.: Benson et al., (2000) Nucleic Acids Res. 28: 15-18).

Kinases and phosphatases regulate many different cell proliferation, differentiation, and signaling processes by adding and removing phosphate groups to and from proteins. Reversible protein phosphorylation is the main strategy for controlling activities of eukaryotic cells. It is estimated that more than 1000 of the 10000 proteins active in a typical mammalian cell are phosphorylated. The high energy phosphate which drives activation is generally transferred from adenosine triphosphate molecules (ATP) to a particular protein by protein kinases and removed from that protein by protein phosphatases. Phosphorylation occurs in response to extracellular signals (hormones, neurotransmitters, growth and differentiation factors, etc.), cell cycle checkpoints, and environmental or nutritional stresses and is roughly analogous to turning on a molecular switch. When the switch goes on, the appropriate protein kinase activates a metabolic enzyme, regulatory protein, receptor, cytoskeletal protein, ion channel or pump, or transcription factor. Uncontrolled signaling has been implicated in a variety of disease conditions including inflammation, cancer, arteriosclerosis, and psoriasis.

### Tyrosine phosphatases:

Protein tyrosine phosphorylation has been shown to be an evolutionary conserved mechanism essential for the regulation of numerous cellular events, including cell growth, tissue differentiation and metabolism (Walton K. M. and Dixon J. E., (1993) Annu Rev Biochem 62:101-120; Li L. and Dixon J. E., (2000) Semin Immunol. 12(1):75-84). Several hundred protein tyrosine phosphatases (herein referred to as PTP) are encoded by the human genome that are subdivided into different classes. Based on subcellular localisation PTPs are classified as receptor-like or intracellular. Receptor-like PTPs contain a transmembrane domain, a receptor-like extracellular domain, and usually two intracellular PTP domains. Only few receptor PTPs contain only one intracellular PTP domain (e.g. Drosophila PTP1 OD and human PTPRB). Characteristics of the extracellular domain are used to further subdivide receptor PTPs into five different types (Mourey R. J. and Dixon, J. E., (1994) Curr Opin Genet Dev 4(1):31-39). Drosophila PTP10D, for example, contains a series of extracellular fibronectin-like repeats and immunoglobulin-like repeats and belongs to Type III receptor PTPs. The only known function of Drosophila PTP10D is an involvement in the control of axon guidance during embryonic development (Sun Q. et al., (2000) Development 127:801-812; Kraut R. et al., (2001) Curr Biol 11:417-430). No function in the regulation of metabolism has been reported for PTP10D or its human homolog.

WO 01/57190 discloses the cDNA sequence of PTPRB (SEQ ID 499), a PTP10D protein, its function as a protein-tyrosine phosphatise, its (recombinant) polypeptide, an antibody thereto, expression vectors, transgenic animals and screening methods.

EP-A-1 046 715 discloses the use of polynucleotides (cDNA: SEQ ID 3) or polypeptides (SEQ ID 4) of human PTP beta (= PTPRB, a PTP10D protein) for diagnosis and inhibition of tumour growth. Also mentioned are antibodies and antisense to human PTP beta. A monitoring assay with human PTP beta and Tie-2 is described where the kinase activity of Tie-2 is measured. Human PTP beta and Tie-2 interact with each other.

So far, it has not been described that PTP10D or the human homologous proteins are involved in the regulation of energy homeostasis and body-weight regulation and related disorders, and thus, no functions in metabolic diseases and other diseases as listed above have been discussed. In this invention we demonstrate that the correct gene dose of PTP10D is essential for maintenance of energy homeostasis. A genetic screen was used to identify that a mutation of a PTP10D homologous gene causes obesity, reflected by a significant increase of triglyceride content, the major energy storage substance.

Polynucleotides encoding a protein with homologies to PTP10D are suitable to investigate diseases and disorders as described above. Further new compositions useful in diagnosis, treatment, and prognosis of diseases and disorders as described above are provided.

Before the present proteins, nucleotide sequences, and methods are described, it is understood that this invention is not limited to the particular methodology, protocols, cell lines, vectors, and reagents described as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are incorporated herein by reference for the purpose of describing and disclosing the cell lines, vectors, and methodologies which are reported in the publications which might be used in connection with the invention. Nothing herein is to be construed as an admission that the invention is not entitled to antedate such disclosure.

The present invention discloses that PTP10D or homologous proteins are regulating the energy homeostasis and fat metabolism especially the metabolism and storage of triglycerides, and polynucleotides, which identify and encode the proteins disclosed in this invention. The invention also relates to vectors, host cells, antibodies, and recombinant methods for producing the polypeptides and polynucleotides of the invention. The invention also relates to the use of these sequences in the diagnosis, study, prevention, and treatment of diseases and disorders, for example, but not limited to, metabolic diseases such as obesity as well as related disorders such as eating disorder, cachexia, diabetes mellitus, hypertension, coronary heart disease, hypercholesterolemia, dyslipidemia, osteoarthritis, gallstones, cancer, e.g. cancers of the reproductive organs, and sleep apnea.

PTP10D or homologous proteins and nucleic acid molecules coding therefore are obtainable from insect or vertebrate species, e.g. mammals or birds. Particularly preferred are homologous nucleic acids, particularly nucleic acids encoding a human RPTP beta protein, a mouse PTP10D like protein, a human tec protein tyrosine kinase protein and mouse protein tyrosine kinase TeciV or a human or mouse EDTP protein.

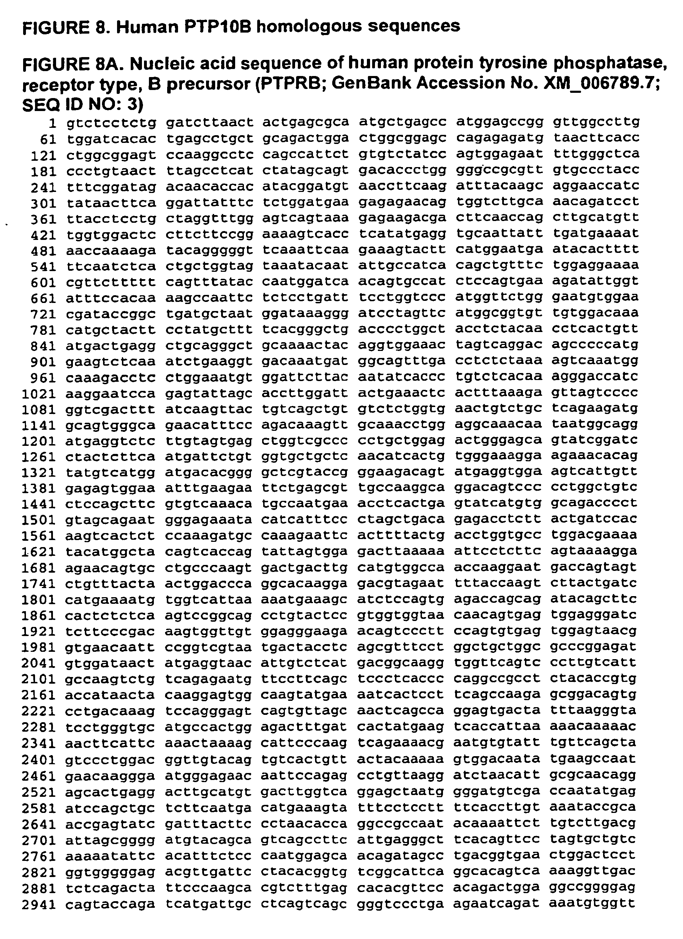

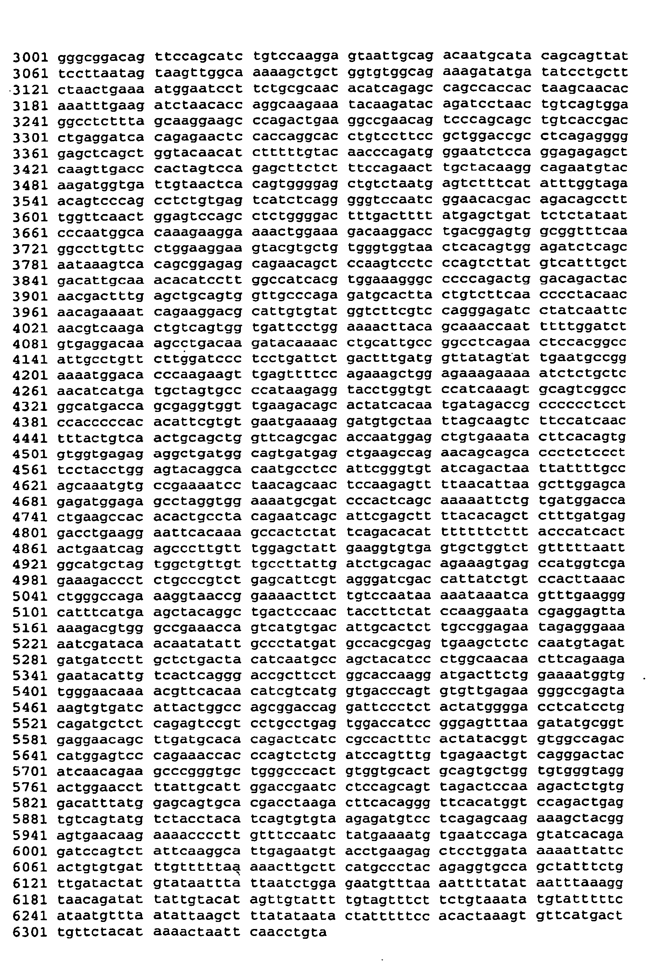

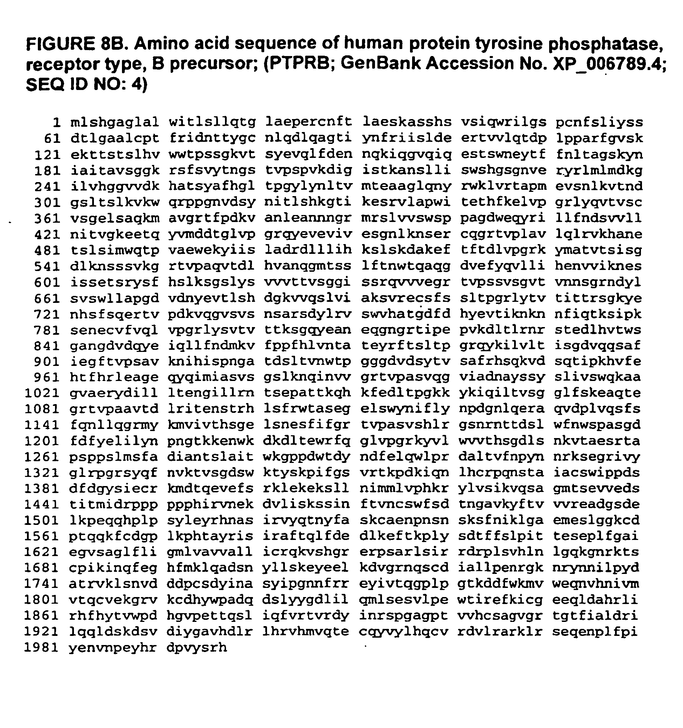

In this invention we particularly refer to protein tyrosin phosphatase 10D (referred to as PTP10D), and PTP10D homologous proteins (for example protein tyrosine phosphatase, receptor type, Beta (PTPRB or RPTP beta) which include Drosophila and mammalian, preferably human, homologous polypeptides or proteins or sequences encoding those proteins as proteins of the invention. Especially preferred embodiments are:
* Drosophila PTP10D (Gadfly Accession Number CG1817), human PTPRB (Genbank Accession Number XM_006789.7 for the cDNA (SEQ ID NO: 3, see Fig. 8A), Genbank Accession Number XP_006789.4 for the protein (SEQ ID NO: 4, see Fig. 8B), and mouse receptor-type protein tyrosine phosphatase (Genbank Accession Number AF157628 for the cDNA, Genbank Accession Number AAF80346 for the protein),

The invention particularly relates to a nucleic acid molecule encoding a polypeptide contributing to regulating the energy homeostasis and the metabolism of triglycerides, wherein said nucleic acid molecule comprises
(a) the nucleotide sequence encoding a protein of the invention and/or a sequence complementary thereto,
(b) a nucleotide sequence which hybridizes at 50°C in a solution containing 1 x SSC and 0.1 % SDS to a sequence of (a),
(c) a sequence corresponding to the sequences of (a) or (b) within the degeneration of the genetic code,
(d) a sequence which encodes a polypeptide which is at least 85%, preferably at least 90%, more preferably at least 95%, more preferably at least 98% and up to 99,6% identical to the amino acid sequence of a protein of the invention,
(e) a sequence which differs from the nucleic acid molecule of (a) to (d) by mutation and wherein said mutation causes an alteration, deletion, duplication and/or premature stop in the encoded polypeptide or
(f) a partial sequence of any of the nucleotide sequences of (a) to (e) having a length of at least 15 bases, preferably at least 20 bases, more preferably at least 25 bases and most preferably at least 50 bases.

The invention is based on the finding that the proteins of the invention and the polynucleotides encoding these, are involved in the regulation of triglyceride storage and therefore energy homeostasis. The invention describes the use of these proteins and polynucleotides or fragments thereof or effectors thereof, e.g. antibodies, aptamers, antisense molecules, RNAi molecules, ribozymes, peptides or low-molecular weight organic compounds or other receptors recognizing the polynucleotide or the polypeptide for the diagnosis, study, prevention, or treatment of diseases and disorders related thereto, including metabolic diseases such as obesity as well as related disorders such as eating disorder, cachexia, diabetes mellitus, hypertension, coronary heart disease, hypercholesterolemia, dyslipidemia, osteoarthritis, gallstones, cancer, e.g. cancers of the reproductive organs, and sleep apnea.

Accordingly, the present invention relates to genes with novel functions in body-weight regulation, energy homeostasis, metabolism, and obesity. To find genes with novel functions in energy homeostasis, metabolism, and obesity, a functional genetic screen was performed with the model organism Drosophila melanogaster (Meigen). Drosophila melanogaster is one of the most intensively studied organisms in biology and serves as a model system for the investigation of many developmental and cellular processes common to higher eukaryotes, including humans (see, for example, Adams et al., (2000) Science 287: 2185-2195). The success of Drosophila melanogaster as a model organism is largely due to the power of forward genetic screens to identify the genes that are involved in a biological process (see, Johnston (2002) Nat Rev Genet 3: 176-188; Rorth, (1996) Proc Natl Acad Sci U S A 93: 12418-12422). One resource for screening was a proprietary Drosophila melanogaster stock collection of EP-lines. The P-vector of this collection has Gal4-UAS-binding sites fused to a basal promoter that can transcribe adjacent genomic Drosophila sequences upon binding of Gal4 to UAS-sites. This enables the EP-line collection for overexpression of endogenous flanking gene sequences. In addition, without activation of the UAS-sites, integration of the EP-element into the gene is likely to cause a reduction of gene activity, and allows determining its function by evaluating the loss-of-function phenotype.

Triglycerides are the most efficient storage for energy in cells, and are significantly increased in obese patients. In this invention, we have used a genetic screen to identify, that mutations of genes encoding the proteins of the invention or homologous genes cause changes in the body weight which is reflected by a significant change in the triglyceride levels. In order to isolate genes with a function in energy homeostasis, several thousand proprietary and publicly available EP-lines were tested for their triglyceride content after a prolonged feeding period (illustrated in more detail in the EXAMPLES). Lines with significantly changed triglyceride content were selected as positive candidates for further analysis.

In this invention, the content of triglycerides of a pool of flies with the same genotype after feeding for six days was analyzed using a triglyceride assay, as, for example, but not for limiting the scope of the invention, is described in more detail below in the examples section. The change of triglyceride content due to the loss of a gene function suggests gene activities in energy homeostasis in a dose dependent manner that controls the amount of energy stored as triglycerides.

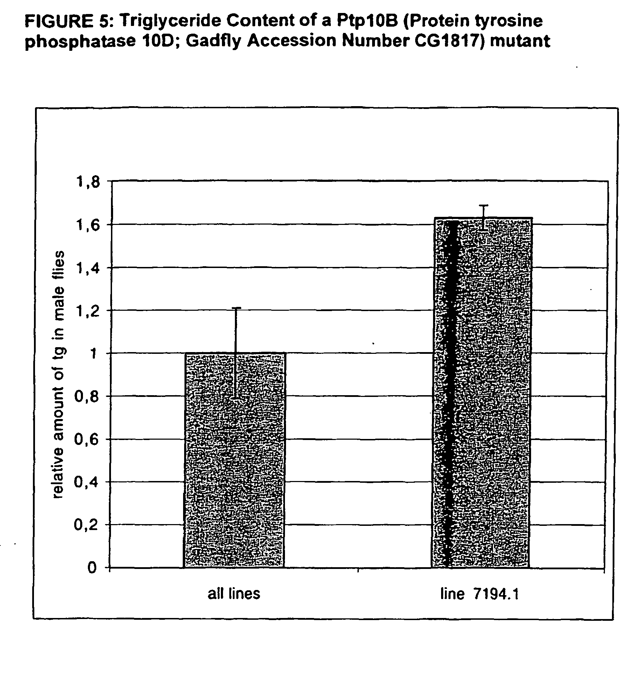

The result of the triglyceride content analysis is shown in FIGURE 5. We found that hemizygous PX7194.1 flies have a higher triglyceride content than the controls (average triglyceride levels). Therefore, the loss of a gene activity in the gene loci, where the EP-vectors are integrated, is responsible for changes in the metabolism of the energy storage triglycerides, therefore representing in all cases an obese fly model. The increase of triglyceride content due to the loss of a gene function suggests gene activities in energy homeostasis in a dose dependent manner that controls the amount of energy stored as triglycerides.

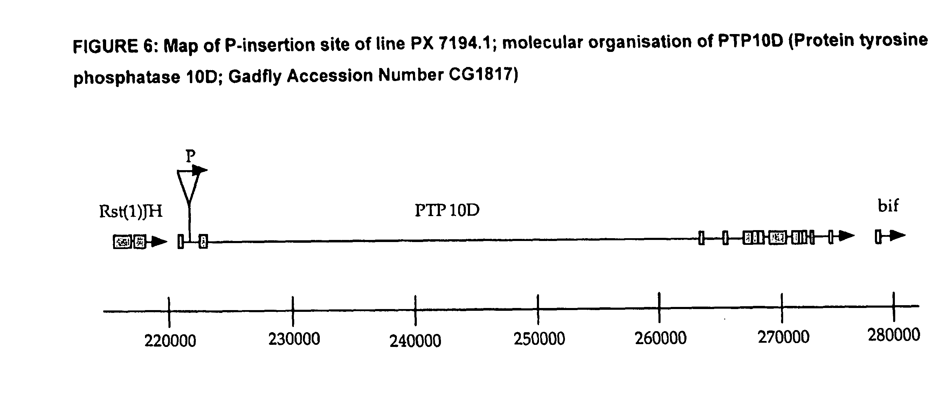

Nucleic acids encoding the proteins of the present invention were identified using a iPCR technique. Genomic DNA sequences were isolated that are localised directly 5' to the EP vectors (herein PX7194.1 integration). Using those isolated genomic sequences public databases like Berkeley Drosophila Genome Project (GadFly, see also FlyBase (1999) Nucleic Acids Research 27:85-88) were screened thereby confirming the homozygous or hemizygous viable or heterozygous integration site of the vectors into the transcription units of the genes FIGURE 6 show the molecular organisation of this gene locus.

The proteins of the invention and homologous proteins and nucleic acid molecules coding therefore are obtainable from insect or vertebrate species, e.g. mammals or birds. Particularly preferred are nucleic acids encoding the human homologs of the proteins of the invention. The present invention is describing polypeptides comprising the amino acid sequences of the proteins of the invention. Comparisons (ClustalX 1.8 analysis or ClustalW 1.82 analysis, see for example Thompson J. D. et al., (1994) Nucleic Acids Res. 22(22):4673-4680; Thompson J. D., (1997) Nucleic Acids Res 25(24):4876-4882; Higgins, D. G. et al., (1996) Methods Enzymol. 266:383-402) between the respective proteins of different species (human and Drosophila) were conducted. Based upon homology, the Drosophila proteins of the invention and each homologous protein or peptide may share at least some activity. No functional data described the regulation of body weight control and related metabolic diseases such as obesity are available in the prior art for the genes claimed in this invention.

Expression profiling studies (see Examples for more detail) confirm the particular relevance of the protein PTPRP as regulator of energy metabolism in mammals.

The invention also encompasses polynucleotides that encode the proteins of the invention. Accordingly, any nucleic acid sequence, which encodes the amino acid sequences of a protein of the invention can be used to generate recombinant molecules that express a protein of the invention. In a particular embodiment, the invention encompasses the polynucleotide encoding a Drosophila or human protein of the invention. It will be appreciated by those skilled in the art that as a result of the degeneracy of the genetic code, a multitude of nucleotide sequences encoding the proteins of the invention, some bearing minimal homology to the nucleotide sequences of any known and naturally occurring gene, may be produced. Thus, the invention contemplates each and every possible variation of nucleotide sequence that could be made by selecting combinations based on possible codon choices. These combinations are made in accordance with the standard triplet genetic code as applied to the nucleotide sequences of naturally occurring proteins of the invention, and all such variations are to be considered as being specifically disclosed. Although nucleotide sequences which encode the proteins of the invention and its variants are preferably capable of hybridising to the nucleotide sequences of the naturally occurring proteins of the invention under appropriately selected conditions of stringency, it may be advantageous to produce nucleotide sequences encoding the proteins of the invention or their derivatives possessing a substantially different codon usage. Codons may be selected to increase the rate at which expression of the peptide occurs in a particular prokaryotic or eukaryotic host in accordance with the frequency with which particular codons are utilised by the host. Other reasons for substantially altering the nucleotide sequence encoding a protein of the invention and its derivatives without altering the encoded amino acid sequences include the production of RNA transcripts having more desirable properties, such as a greater half-life, than transcripts produced from the naturally occurring sequences. The invention also encompasses production of DNA sequences, or portions thereof, which encode the proteins of the invention and their derivatives, entirely by synthetic chemistry. After production, the synthetic sequence may be inserted into any of the many available expression vectors and cell systems using reagents that are well known in the art at the time of the filing of this application. Moreover, synthetic chemistry may be used to introduce mutations into a sequence encoding a protein of the invention or any portion thereof.

Also encompassed by the invention are polynucleotide sequences that are capable of hybridising to the claimed nucleotide sequences, and in particular, those of the polynucleotide encoding a protein of the invention under various conditions of stringency. Hybridisation conditions are based on the melting temperature (Tm) of the nucleic acid binding complex or probe, as taught in Wahl, G. M. and S. L. Berger (1987: Methods Enzymol. 152:399-407) and Kimmel, A. R. (1987; Methods Enzymol. 152:507-511), and may be used at a defined stringency. Preferably, hybridization under stringent conditions means that after washing for 1 h with 1 x SSC and 0.1 % SDS at 50°C, preferably at 55°C, more preferably at 62°C and most preferably at 68°C, particularly for 1 h in 0.2 x SSC and 0.1 % SDS at 50°C, preferably at 55°C, more preferably at 62°C and most preferably at 68°C, a positive hybridization signal is observed. Altered nucleic acid sequences encoding the proteins of the invention which are encompassed by the invention include deletions, insertions, or substitutions of different nucleotides resulting in a polynucleotide that encodes the same or a functionally equivalent protein of the invention.

The encoded proteins may also contain deletions, insertions, or substitutions of amino acid residues, which produce a silent change and result in a functionally equivalent protein of the invention. Deliberate amino acid substitutions may be made on the basis of similarity in polarity, charge, solubility, hydrophobicity, hydrophilicity, and/or the amphipathic nature of the residues as long as the biological activity of the proteins of the invention is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine; glycine and alanine; asparagine and glutamine; serine and threonine; phenylalanine and tyrosine. Furthermore, the invention relates to peptide fragments of the proteins or derivatives thereof such as cyclic peptides, retro-inverso-peptides or peptide mimetics having a length of at least 4, preferably at least 6 and up to 50 amino acids.

Also included within the scope of the present invention are alleles of the genes encoding the proteins of the invention. As used herein, an "allele" or "allelic sequence" is an alternative form of the gene, which may result from at least one mutation in the nucleic acid sequence. Alleles may result in altered mRNAs or polypeptides whose structures or function may or may not be altered. Any given gene may have none, one, or many allelic forms. Common mutational changes, which give rise to alleles, are generally ascribed to natural deletions, additions, or substitutions of nucleotides. Each of these types of changes may occur alone, or in combination with the others, one or more times in a given sequence.

The nucleic acid sequences encoding the proteins of the invention may be extended utilising a partial nucleotide sequence and employing various methods known in the art to detect upstream sequences such as promoters and regulatory elements. For example, one method which may be employed, "restriction-site" PCR, uses universal primers to retrieve unknown sequence adjacent to a known locus (Sarkar, G. (1993) PCR Methods Applic. 2:318-322). Inverse PCR may also be used to amplify or extend sequences using divergent primers based on a known region (Triglia, T. et al. (1988) Nucleic Acids Res. 16:8186).

Another method which may be used is capture PCR which involves PCR amplification of DNA fragments adjacent to a known sequence in human and yeast artificial chromosome DNA (Lagerstrom, M. et al. (PCR Methods Applic. 1:111-119). Another method which may be used to retrieve unknown sequences is that of Parker, J. D. et al. (1991; Nucleic Acids Res. 19:3055-3060). Additionally, one may use PCR, nested primers, and PROMOTERFINDER libraries to walk in genomic DNA (Clontech, Palo Alto, Calif.). This process avoids the need to screen libraries and is useful in finding intron/exon junctions.

In order to express biologically active proteins of the invention, the nucleotide sequences encoding the proteins of the invention or functional equivalents, optionally in the form of fusion proteins, may be inserted into appropriate expression vectors, i.e., a vector, which contains the necessary elements for the transcription and translation of the inserted coding sequence. Methods, which are well known to those skilled in the art, may be used to construct expression vectors containing sequences encoding a protein of the invention and appropriate transcriptional and translational control elements. These methods include in vitro recombinant DNA techniques. synthetic techniques, and in vivo genetic recombination. Such techniques are described in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York, N.Y.

Regulatory elements include for example a promoter, an initiation codon, a stop codon, a mRNA stability regulatory element, and a polyadenylation signal. Expression of a polynucleotide can be assured by (i) constitutive promoters such as the Cytomegalovirus (CMV) promoter/enhancer region, (ii) tissue specific promoters such as the insulin promoter (see, Soria et al., 2000, Diabetes 49:157), SOX2 gene promotor (see Li et al., 1998, Curr. Biol. 8:971-4), Msi-1 promotor (see Sakakibara et al., 1997, J. Neuroscience 17:8300-8312), alpha-cardia myosin heavy chain promotor or human atrial natriuretic factor promotor (Klug et al., 1996, J. clin. Invest 98:216-24; Wu et al., 1989, J. Biol. Chem. 264:6472-79)or (iii) inducible promoters such as the tetracycline inducible system. Expression vectors can also contain a selection agent or marker gene that confers antibiotic resistance such as the neomycin, hygromycin or puromycin resistance genes. These methods include in vitro recombinant DNA techniques, synthetic techniques, and in vivo genetic recombination. Such techniques are described in Sambrook, J. et al. (1989) Molecular Cloning, A Laboratory Manual, Cold Spring Harbor Press, Plainview, N.Y. and Ausubel, F.M. et al. (1989) Current Protocols in Molecular Biology, John Wiley & Sons, New York, N.Y. In a further embodiment of the invention, natural, modified or recombinant nucleic acid sequences encoding the proteins of the invention and homologous proteins may be ligated to a heterologous sequence to encode a fusion protein.

A variety of expression vector/host systems may be utilised to contain and express sequences encoding the proteins of the invention. These include, but are not limited to, micro-organisms such as bacteria transformed with recombinant bacteriophage, plasmid, or cosmid DNA expression vectors; yeast transformed with yeast expression vectors; insect cell systems infected with virus expression vectors (e.g., baculovirus, adenovirus, adeno-associated virus, lentivirus, retrovirus); plant cell systems transformed with virus expression vectors (e.g., cauliflower mosaic virus, CaMV; tobacco mosaic virus, TMV) or with bacterial expression vectors (e.g., Ti or PBR322 plasmids); or animal cell systems.

The presence of polynucleotide sequences encoding the proteins of the invention can be detected by DNA-DNA or DNA-RNA hybridisation and/or amplification using probes or portions or fragments of polynucleotides encoding a protein of the invention. Nucleic acid amplification based assays involve the use of oligonucleotides or oligomers based on the sequences encoding said protein of the invention to detect transformants containing DNA or RNA encoding said protein of the invention. As used herein "oligonucleotides" or "oligomers" refer to a nucleic acid sequence of at least about 10 nucleotides and as many as about 60 nucleotides, preferably about 15 to 30 nucleotides, and more preferably about 20-25 nucleotides, which can be used as a probe or amplimer.

A variety of protocols for detecting and measuring the expression of the proteins of the invention, using either polyclonal or monoclonal antibodies specific are known in the art. Examples include enzyme-linked immunosorbent assay (ELISA), radioimmunoassay (RIA), and fluorescence activated cell sorting (FACS). A two-site, monoclonal-based immunoassay utilising monoclonal antibodies reactive to two non-interfering epitopes on the proteins of the invention is preferred, but a competitive binding assay may be employed. These and other assays are described, among other places, in Hampton, R. et al. (1990; Serological Methods, a Laboratory Manual, APS Press, St Paul, Minn.) and Maddox, D. E. et al. (1983; J. Exp. Med. 158:1211-1216).

A wide variety of labels and conjugation techniques are known by those skilled in the art and may be used in various nucleic acid and amino acid assays. Means for producing labelled hybridisation or PCR probes for detecting sequences related to polynucleotides encoding a protein of the invention include oligo-labelling, nick translation, end-labelling or PCR amplification using a labelled nucleotide, or enzymatic synthesis. These procedures may be conducted using a variety of commercially available kits (Pharmacia & Upjohn, (Kalamazoo, Mich.); Promega (Madison Wis.); and U.S. Biochemical Corp., (Cleveland, Ohio).

Suitable reporter molecules or labels, which may be used, include radionuclides, enzymes, fluorescent, chemiluminescent, or chromogenic agents as well as substrates, co-factors, inhibitors, magnetic particles, and the like.

Host cells transformed with nucleotide sequences encoding the proteins of the invention may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides which encode the proteins of the invention may be designed to contain signal sequences, which direct secretion of the proteins of the invention through a prokaryotic or eukaryotic cell membrane. Other recombinant constructions may be used to join sequences encoding the proteins of the invention to nucleotide sequence encoding a polypeptide domain, which will facilitate purification of soluble proteins. Such purification facilitating domains include, but are not limited to, metal chelating peptides such as histidine-tryptophan modules that allow purification on immobilised metals, protein A domains that allow purification on immobilised immunoglobulin, and the domain utilised in the FLAG extension/affinity purification system (Immunex Corp., Seattle, Wash.) The inclusion of cleavable linker sequences such as those specific for Factor XA or Enterokinase (Invitrogen, San Diego, Calif.) between the purification domain and the proteins of the invention may be used to facilitate purification. One such expression vector provides for expression of a fusion protein containing the proteins of the invention and a nucleic acid encoding 6 histidine residues preceding a Thioredoxine or an Enterokinase cleavage site. The histidine residues facilitate purification on IMIAC (immobilised metal ion affinity chromatography as described in Porath, J. et al. (1992, Prot. Exp. Purif. 3: 263-281)) while the Enterokinase cleavage site provides a means for purifying the proteins of the invention from the fusion protein. A discussion of vectors which contain fusion proteins is provided in Kroll, D. J. et al. (1993; DNA Cell Biol. 12:441-453). In addition to recombinant production, fragments of the proteins of the invention may be produced by direct peptide synthesis using solid-phase techniques (Merrifield J. (1963) J. Am. Chem. Soc. 85:2149-2154). Protein synthesis may be performed using manual techniques or by automation. Automated synthesis may be achieved, for example, using Applied Biosystems 431 A peptide synthesiser (Perkin Elmer). Various fragments of the proteins of the invention may be chemically synthesised separately and combined using chemical methods to produce the full length molecule.

### Therapeutics

The data disclosed in this invention show that the nucleic acids and proteins of the invention and effectors thereof are useful in therapeutic applications implicated in metabolic disorders such as obesity as well as related disorders such as eating disorder, cachexia, diabetes mellitus, hypertension, coronary heart disease, hypercholesterolemia, dyslipidemia. Hence, therapeutic uses for the nucleic acids and proteins of the invention are, for example but not limited to, the following: (i) protein therapeutic, (iii) antibody target (therapeutic, drug targeting/cytotoxic antibody), (v) gene therapy (gene delivery/gene ablation), and (vii) tissue regeneration in vitro and in vivo (regeneration for all these tissues and cell types composing these tissues and cell types derived from these tissues).

The nucleic acids and proteins of the invention are useful in therapeutic applications implicated in various applications as described below. For example, but not limited to, cDNAs encoding the proteins of the invention and particularly their human homologues may be useful in gene therapy, and the proteins of the invention and particularly their human homologues may be useful when administered to a subject in need thereof. By way of non-limiting example, the compositions of the present invention will have efficacy for treatment of patients suffering from, for example, but not limited to, in metabolic disorders as described above.

For example, in one aspect, antibodies which are specific for a protein of the invention may be used directly as an antagonist, or indirectly as a targeting or delivery mechanism for bringing a pharmaceutical agent to cells or tissue which express said protein of the invention. The antibodies may be generated using methods that are well known in the art. Such antibodies may include, but are not limited to, polyclonal, monoclonal, chimerical, single chain, Fab fragments, and fragments produced by a Fab expression library. Neutralising antibodies, (i.e., those which inhibit dimer formation) are especially preferred for therapeutic use.

For the production of antibodies, various hosts including goats, rabbits, rats, mice, humans, and others, may be immunised by injection with a protein of the invention, or any fragment or oligopeptide thereof which has immunogenic properties. Depending on the host species, various adjuvants may be used to increase immunological response. It is preferred that the peptides, fragments, or oligopeptides used to induce antibodies to a protein of the invention have an amino acid sequence consisting of at least five amino acids, and more preferably at least 10 amino acids.

Monoclonal antibodies to a protein of the invention may be prepared using any technique which provides for the production of antibody molecules by continuous cell lines in culture. These include, but are not limited to, the hybridoma technique, the human B-cell hybridoma technique, and the EBV-hybridoma technique (Köhler, G. et al. (1975) Nature 256:495-497; Kozbor, D. et al. (1985) J. Immunol. Methods 81:31-42; Cote, R. J. et al. Proc. Natl. Acad. Sci. 80:2026-2030; Cole, S. P. et al. (1984) Mol. Cell Biol. 62:109-120).

In addition, techniques developed for the production of "chimeric antibodies", the splicing of mouse antibody genes to human antibody genes to obtain a molecule with appropriate antigen specificity and biological activity can be used (Morrison, S. L. et al. (1984) Proc. Natl. Acad. Sci. 81:6851-6855; Neuberger, M. S. et al (1984) Nature 312:604-608; Takeda, S. et al. (1985) Nature 314:452-454). Alternatively, techniques described for the production of single chain antibodies may be adapted, using methods known in the art, to produce single chain antibodies specific for a protein of the invention. Antibodies with related specificity, but of distinct idiotypic composition, may be generated by chain shuffling from random combinatorial immunoglobulin libraries (Burton, D. R. (1991) Proc. Natl. Acad. Sci. 88:11120-3). Antibodies may also be produced by inducing in vivo production in the lymphocyte population or by screening recombinant immunoglobulin libraries or panels of highly specific binding reagents as disclosed in the literature (Orlandi, R. et al. (1989) Proc. Natl. Acad. Sci. 86:3833-3837; Winter, G. et al. (1991) Nature 349:293-299).

Antibody fragments, which contain specific binding sites for a protein of the invention, may also be generated. For example, such fragments include, but are not limited to, the F(ab')₂ fragments which can be produced by Pepsin digestion of the antibody molecule and the Fab fragments which can be generated by reducing the disulfide bridges of F(ab')₂ fragments. Alternatively, Fab expression libraries may be constructed to allow rapid and easy identification of monoclonal Fab fragments with the desired specificity (Huse, W. D. et al. (1989) Science 254:1275-1281).

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between a protein of the invention and its specific antibody. A two-site, monoclonal-based immunoassay utilising monoclonal antibodies reactive to two non-interfering epitopes of a protein of the invention is preferred, but a competitive binding assay may also be employed (Maddox, supra).

In another embodiment of the invention, the polynucleotides encoding the proteins of the invention, or any fragment thereof, or nucleic acid effector molecules such as antisense molecules, aptamers, RNAi molecules or ribozymes may be used for therapeutic purposes. In one aspect, aptamers, i.e. nucleic acid molecules, which are capable of binding to a protein of the invention and modulating its activity may be generated by a screening and selection procedure involving the use of combinatorial nucleic acid libraries.

In a further aspect, antisense molecules to the polynucleotide encoding a protein of the invention may be used in situations in which it would be desirable to block the transcription of the mRNA. In particular, cells may be transformed with sequences complementary to polynucleotides encoding a protein of the invention. Thus, antisense molecules may be used to modulate the activity of the proteins of the invention, or to achieve regulation of gene function. Such technology is now well know in the art, and sense or antisense oligomers or larger fragments, can be designed from various locations along the coding or control regions of sequences encoding the proteins of the invention. Expression vectors derived from retroviruses, adenovirus, herpes or vaccinia viruses, or from various bacterial plasmids may be used for delivery of nucleotide sequences to the targeted organ, tissue or cell population. Methods, which are well known to those skilled in the art, can be used to construct recombinant vectors, which will express antisense molecules complementary to the polynucleotides of the gene encoding a protein of the invention. These techniques are described both in Sambrook et al. (supra) and in Ausubel et al. (supra). Genes encoding the proteins of the invention can be turned off by transforming a cell or tissue with expression vectors which express high levels of polynucleotide or fragment thereof which encodes a protein of the invention. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector and even longer if appropriate replication elements are part of the vector system.

As mentioned above, modifications of gene expression can be obtained by designing antisense molecules, DNA, RNA, or PNA, to the control regions of the gene encoding a protein of the invention, i.e., the promoters, enhancers, and introns. Oligonucleotides derived from the transcription initiation site, e.g., between positions -10 and +10 from the start site, are preferred. Similarly, inhibition can be achieved using "triple helix" base-pairing methodology. Triple helix pairing is useful because it cause inhibition of the ability of the double helix to open sufficiently for the binding of polymerases, transcription factors, or regulatory molecules. Recent therapeutic advances using triplex DNA have been described in the literature (Gee, J. E. et al. (1994) In; Huber, B. E. and B. I. Carr, Molecular and Immunologic Approaches, Futura Publishing Co., Mt. Kisco, N.Y.). The antisense molecules may also be designed to block translation of mRNA by preventing the transcript from binding to ribosomes.

Ribozymes, enzymatic RNA molecules, may also be used to catalyse the specific cleavage of RNA. The mechanism of ribozyme action involves sequence-specific hybridisation of the ribozyme molecule to complementary target RNA, followed by endonucleolytic cleavage. Examples, which may be used, include engineered hammerhead motif ribozyme molecules that can be specifically and efficiently catalyse endonucleolytic cleavage of sequences encoding a protein of the invention. Specific ribozyme cleavage sites within any potential RNA target are initially identified by scanning the target molecule for ribozyme cleavage sites which include the following sequences: GUA, GUU, and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibility to hybridisation with complementary oligonucleotides using ribonuclease protection assays.

Nucleic acid effector molecules, e.g. antisense molecules and ribozymes of the invention may be prepared by any method known in the art for the synthesis of nucleic acid molecules. These include techniques for chemically synthesising oligonucleotides such as solid phase phosphoramidite chemical synthesis. Alternatively, RNA molecules may be generated by in vitro and in vivo transcription of DNA sequences encoding the proteins of the invention. Such DNA sequences may be incorporated into a variety of vectors with suitable RNA polymerase promoters such as T7 or SP6. Alternatively, these cDNA constructs that synthesise antisense RNA constitutively or inducibly can be introduced into cell lines, cells, or tissues. RNA molecules may be modified to increase intracellular stability and half-life. Possible modifications include, but are not limited to, the addition of flanking sequences at the 5' and/or 3' ends of the molecule or the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine, and wybutosine, as well as acetyl-, methyl-, thio-, and similarly modified forms of adenine, cytidine, guanine, thymine, and uridine which are not as easily recognised by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection and by liposome injections may be achieved using methods, which are well known in the art. Any of the therapeutic methods described above may be applied to any suitable subject including, for example, mammals such as dogs, cats, cows, horses, rabbits, monkeys, and most preferably, humans.

An additional embodiment of the invention relates to manufacture of a medicament for the administration of a pharmaceutical composition, in conjunction with a pharmaceutically acceptable carrier, for any of the therapeutic effects discussed above. Such pharmaceutical compositions may consist of a protein of the invention, antibodies to a protein of the invention, mimetics, agonists, antagonists, or inhibitors of a protein of the invention. The compositions may be administered alone or in combination with at least one other agent, such as stabilising compound, which may be administered in any sterile, biocompatible pharmaceutical carrier, including, but not limited to, saline, buffered saline, dextrose, and water. The compositions may be administered to a patient alone, or in combination with other agents, drugs or hormones. The pharmaceutical compositions utilised in this invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries, which facilitate processing of the active compounds into preparations which, can be used pharmaceutically. Further details on techniques for formulation and administration may be found in the latest edition of Remington's Pharmaceutical Sciences (Maack Publishing Co., Easton, Pa.). Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilising processes. After pharmaceutical compositions have been prepared, they can be placed in an appropriate container and labelled for treatment of an indicated condition. For administration of the proteins of the invention, such labelling would include amount, frequency, and method of administration.

Pharmaceutical compositions suitable for use in the invention include compositions wherein the active ingredients are contained in an effective amount to achieve the intended purpose. The determination of an effective dose is well within the capability of those skilled in the art. For any compounds, the therapeutically effective does can be estimated initially either in cell culture assays, e.g., of preadipocyte cell lines, or in animal models, usually mice, rabbits, dogs, or pigs. The animal model may also be used to determine the appropriate concentration range and route of administration. Such information can then be used to determine useful doses and routes for administration in humans. A therapeutically effective dose refers to that amount of active ingredient, for example a nucleic acid or protein of the invention, or fragments thereof, or antibodies, which are sufficient for treating a specific condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions, which exhibit large therapeutic indices, are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage from employed, sensitivity of the patient, and the route of administration. The exact dosage will be determined by the practitioner, in light of factors related to the subject that requires treatment. Dosage and administration are adjusted to provide sufficient levels of the active moiety or to maintain the desired effect. Factors, which may be taken into account, include the severity of the disease state, general health of the subject, age, weight, and gender of the subject, diet, time and frequency of administration, drug combination(s), reaction sensitivities, and tolerance/response to therapy.

Long-acting pharmaceutical compositions may be administered every 3 to 4 days, every week, or once every two weeks depending on half-life and clearance rate of the particular formulation. Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art employ different formulations for nucleotides than for proteins or their inhibitors. Similarly, delivery of polynucleotides or polypeptides will be specific to particular cells, conditions, locations, etc.

The nucleic acids encoding the proteins of the invention can be used to generate transgenic cell lines and animals. These transgenic non-human animals are useful in the study of the function and regulation of the proteins of the invention in vivo. Transgenic animals, particularly mammalian transgenic animals, can serve as a model system for the investigation of many developmental and cellular processes common to humans. A variety of non-human models of metabolic disorders can be used to test modulators of the protein of the invention. Misexpression (for example, overexpression or lack of expression) of the protein of the invention, particular feeding conditions, and/or administration of biologically active compounts can create models of metablic disorders.

In one embodiment of the invention, such assays use mouse models of insulin resistance and/or diabetes, such as mice carrying gene knockouts in the leptin pathway (for example, ob (leptin) or db (leptin receptor) mice). Such mice develop typical symptoms of diabetes , show hepatic lipid accumulation and frequently have increased plasma lipid levels (see Bruning et al, 1998, Mol. Cell. 2:449-569). Susceptible wild type mice (for example C57BI/6) show similiar symptoms if fed a high fat diet. In addition to testing the expression of the proteins of the invention in such mouse strains (see EXAMPLE 4), these mice could be used to test whether administration of a candidate modulator alters for example lipid accumulation in the liver, in plasma, or adipose tissues using standard assays well known in the art, such as FPLC, colorimetric assays, blood glucose level tests, insulin tolerance tests and others.

Transgenic animals may be made through homologous recombination in embryonic stem cells, where the normal locus of the gene encoding the protein of the invention is mutated. Alternatively, a nucleic acid construct encoding the protein is injected into oocytes and is randomly integrated into the genome. One may also express the genes of the invention or variants thereof in tissues where they are not normally expressed or at abnormal times of development. Furthermore, variants of the genes of the invention like specific constructs expressing anti-sense molecules or expression of dominant negative mutations, which will block or alter the expression of the proteins of the invention may be randomly integrated into the genome. A detectable marker, such as lac Z or luciferase may be introduced into the locus of the genes of the invention, where upregulation of expression of the genes of the invention will result in an easily detectable change in phenotype. Vectors for stable integration include plasmids, retroviruses and other animal viruses, yeast artificial chromosomes (YACs), and the like. DNA constructs for homologous recombination will contain at least portions of the genes of the invention with the desired genetic modification, and will include regions of homology to the target locus. Conveniently, markers for positive and negative selection are included. DNA constructs for random integration do not need to contain regions of homology to mediate recombination. DNA constructs for random integration will consist of the nucleic acids encoding the proteins of the invention, a regulatory element (promoter), an intron and a poly-adenylation signal. Methods for generating cells having targeted gene modifications through homologous recombination are known in the field. For embryonic stem (ES) cells, an ES cell line may be employed, or embryonic cells may be obtained freshly from a host, e.g. mouse, rat, guinea pig, etc. Such cells are grown on an appropriate fibroblast-feeder layer and are grown in the presence of leukemia inhibiting factor (LIF). ES or embryonic cells may be transfected and can then be used to produce transgenic animals. After transfection, the ES cells are plated onto a feeder layer in an appropriate medium. Cells containing the construct may be selected by employing a selection medium. After sufficient time for colonies to grow, they are picked and analyzed for the occurrence of homologous recombination. Colonies that are positive may then be used for embryo manipulation and morula aggregation. Briefly, morulae are obtained from 4 to 6 week old superovulated females, the Zona Pellucida is removed and the morulae are put into small depressions of a tissue culture dish. The ES cells are trypsinized, and the modified cells are placed into the depression closely to the morulae. On the following day the aggregates are transfered into the uterine horns of pseudopregnant females. Females are then allowed to go to term. Chimeric offsprings can be readily detected by a change in coat color and are subsequently screened for the transmission of the mutation into the next generation (F1-generation). Offspring of the F1-generation are screened for the presence of the modified gene and males and females having the modification are mated to produce homozygous progeny. If the gene alterations cause lethality at some point in development, tissues or organs can be maintained as allogenic or congenic grafts or transplants, or in vitro culture. The transgenic animals may be any non-human mammal, such as laboratory animal, domestic animals, etc., for example, mouse, rat, guinea pig, sheep, cow, pig, and others. The transgenic animals may be used in functional studies, drug screening, and other applications and are useful in the study of the function and regulation of the proteins of the invention in vivo.

The Figures show:
Figure 5 shows the relative triglyceride content (triglyceride/weight) of Drosophila PTP1OB (GadFly Accession Number CG 1817) mutants. Shown is the change of triglyceride of PX7194.1 flies caused by hemizygous viable integration of the P-vector into the annotated transcription unit (column 2) in comparison to controls containing all fly lines of the PX mutant collection ('all lines', column 1).
Figure 6 shows the molecular organisation of the mutated PTP10D gene locus. In the map of the P-insertion site of line PX7194.1, PTP10D referres to Receptor Protein Tyrosine Phosphatase 10D encoding gene, bif referres to bifocal, and Rst(1)JH referrs to Resistance to Juvenile Hormone.

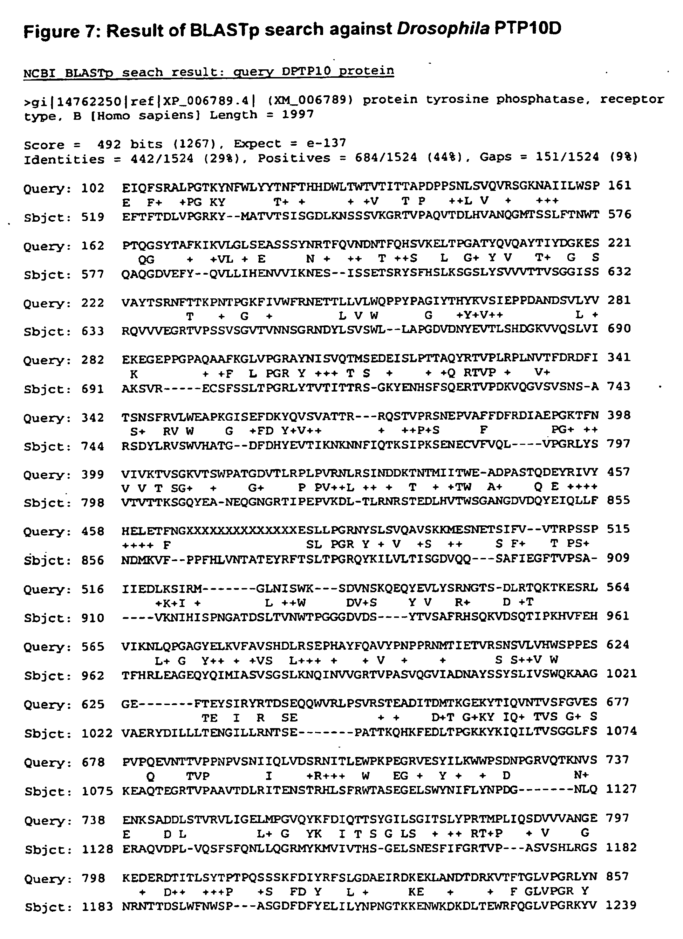

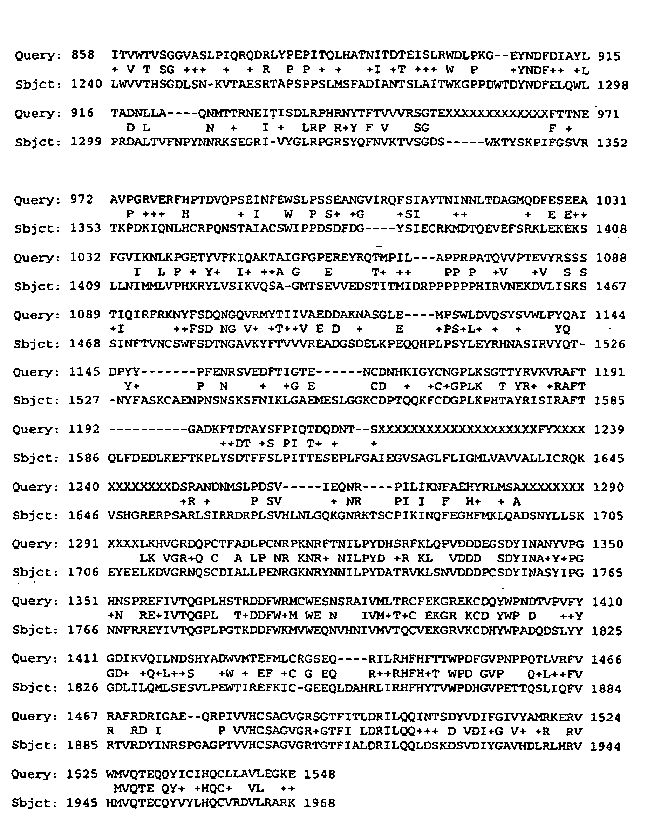

Figure 7 shows the results of a BLASTp search against Drosophila PTP10D.
Figure 8 shows the human PTP10D homologous sequences
Figure 8A shows the nucleic acid sequence of human protein tyrosine phosphatase, receptor type, B (PTPRB) (SEQ ID NO: 3).
Figure 8B shows the amino acid sequence (one-letter code) of human protein tyrosine phosphatase, receptor type, B (PTPRB) (SEQ ID NO: 4).
Figure 9 shows the expression of the PTPRB gene in mammalian tissues.
Figure 9A shows the real-time PCR analysis of PTPRB expression in wildtype mouse tissues. The relative RNA-expression is shown on the Y-axis, the tissues tested are given on the X-axis. WAT = white adipose tissue, BAT = brown adipose tissue.
Figure 9B shows the real-time PCR analysis of PTPRB expression in different tissues of wildtype mice (wt-mice), of fasted mice (fasted-mice), and of genetically obese mice (ob/ob-mice). The relative RNA-expression is shown on the Y-axis, the tissues tested are given on the X-axis. WAT = white adipose tissue, BAT = brown adipose tissue.

The examples illustrate the invention:

### Example 1: Measurement of triglyceride content in Drosophila

The change of triglyceride content of Drosophila melanogaster containing a special expression system (EP-element; Rorth P., (1996) Proc Natl Acad Sci USA 93(221:12418-12422) was measured. Mutant flies are obtained from fly mutation stock collections (proprietary fly mutation stock collection; P insertion Mutation Stock Center, Sezged, Hungary). The flies are grown under standard conditions known to those skilled in the art. In the course of the experiment, additional feedings with bakers yeast (Saccharomyces cerevisiae) are provided. The average increase of triglyceride content of Drosophila containing the PX7194.1, vector in hemizygous viable integration was investigated in comparison to control flies (FIGURE 5). For determination of triglyceride, flies were incubated for 5 min at 90°C in an aqueous buffer using a waterbath, followed by hot extraction. After another 5 min incubation at 90°C and mild centrifugation, the triglyceride content of the flies extract was determined using Sigma Triglyceride (INT 336-10 or -20) assay by measuring changes in the optical density according to the manufacturer's protocol. As a reference protein content of the same extract was measured using BIO-RAD DC Protein Assay according to the manufacturer's protocol, or the weight of the flies was measured. The assays were repeated three times. The average triglyceride level of all flies of the PX collection (referred to as 'all lines' in FIGURE 5) is shown as 100% in FIGURE 5.

PX7194.1 hemizygous flies show constantly a higher triglyceride content than the controls (approx. 60%; column 2 in FIGURE 5). Therefore, the change of gene activity in the locus of the PX7194.1 integration on chromosome X where the PX-vector of PX7194.1 flies is hemizygous viable integrated, is responsible for changes in the metabolism of the energy storage triglycerides.

The increase of triglyceride content due to the potential loss of a gene function suggests potential gene activities in energy homeostasis in a dose dependent manner that controls the amount of energy stored as triglycerides.

### Example 2: Identification of Drosophila genes responsible for changes in triglyceride levels

Using the iPCR method, genomic DNA sequences were isolated that are localised directly adjacent in 5' direction of the integration site of the EP vectors (herein PX7194.1). Using those isolated genomic sequences public databases like Berkeley Drosophila Genome Project (GadFly) were screened thereby identifying the integration sites of the vectors. FIGURE 6 shows the molecular organisation of this gene locus.

The chromosomal localization site of the integration of the vector in line PX7194.1 is at gene locus X, 10C7-D1. In FIGURE 6, numbers represent the coordinates of the genomic DNA (starting at position 220000 on chromosome X, ending at position 280000 on chromosome X). Grey bars predicted exons of the genes (as predicted by the Berkeley Drosophila Genome Project, GadFly and by Magpie). The integration site of the vector for line PX7194.1 is indicated at position 222666. PTP10D encodes for a gene that has been identified and cloned by Tian,S et al. (1991) (1991) Cell 67:675 and Yang,X. et al. (1991) Cell 67:661. Using the published sequences of the PTP10D encoding gene, the hemizygous viable integration site of the PX7194.1 vector has been determined. It is located in an intron between the first and second exon of the PTP10D gene (5' to the start codon), causing an increase of triglyceride content.

### Example 3: Identification of human PTP10D, homologues

The proteins of the invention and homologous proteins and nucleic acid molecules coding therefore are obtainable from insect or vertebrate species, e.g. mammals or birds. Particularly preferred are nucleic acids encoding the Drosophila and human homologs of the proteins of the invention. Sequences homologous to Drosophila proteins of the invention were identified using the publicly available program BLASTP 2.2.3 of the non-redundant protein data base of the National Center for Biotechnology Information (NCBI) (see, Altschul et. Al., (1997), Nucleic Acids Res. 25:3389-2402).

Particularly preferred are nucleic acids encoding Drosophila PTP1 OD (Gadfly Accession Number CG 1817), human PTPRB (Genbank Accession Number XM_006789.7 for the cDNA (SEQ ID NO: 3, see Fig. 8A), Genbank Accession Number XP_006789.4 for the protein (SEQ ID NO: 4, see Fig. 8B), and mouse receptor-type protein tyrosine phosphatase (Genbank Accession Number AF157628 for the cDNA, Genbank Accession Number AAF80346 for the protein).

### Example 4: Expression of the polypeptides in mammalian (mouse) tissues

For analyzing the expression of the polypeptides disclosed in this invention in mammalian tissues, several mouse strains (preferably mice strains C57BI/6J, C57BI/6 ob/ob and C57BI/KS db/db which are standard model systems in obesity and diabetes research) were purchased from Harlan Winkelmann (33178 Borchen, Germany) and maintained under constant temperature (preferably 22°C), 40 per cent humidity and a light / dark cycle of preferably 14 / 10 hours. The mice were fed a standard chow (for example, from ssniff Spezialitäten GmbH, order number ssniff M-Z V1126-000). For the fasting experiment ("fasted wild type mice"), wild type mice were starved for 48 h without food, but only water supplied ad libitum. (see, for example, Schnetzler et al. J Clin Invest 1993 Jul;92(1):272-80, Mizuno et al. Proc Natl Acad Sci U S A 1996 Apr 16;93(8):3434-8). Animals were sacrificed at an age of 6 to 8 weeks. The animal tissues were isolated according to standard procedures known to those skilled in the art, snap frozen in liquid nitrogen and stored at -80°C until needed.

For analyzing the role of the proteins disclosed in this invention in the in vitro differentiation of different mammalian cell culture cells for the conversion of pre-adipocytes to adipocytes, mammalian fibroblast (3T3-L1) cells (e.g., Green & Kehinde, Cell 1: 113-116, 1974) were obtained from the American Tissue Culture Collection (ATCC, Hanassas, VA, USA; ATCC- CL 173). 3T3-L1 cells were maintained as fibroblasts and differentiated into adipocytes as described in the prior art (e.g., Qiu. et al., J. Biol. Chem. 276:11988-95, 2001; Slieker et al., BBRC 251: 225-9, 1998). In brief, cells were plated in DMEM/10% FCS (invitrogen, Karlsruhe, Germany) at 50,000 cells/well in duplicates in 6-well plastic dishes and cultured in a humidified atmosphere of 5% CO₂ at 37°C. At confluence (defined as day 0: d0) cells were transferred to serum-free (SF) medium, containing DMEM/HamF12 (3:1; Invitrogen), Fetuin (300*µ*g/ml; Sigma, Munich, Germany), transferrin (2*µ*g/ml; Sigma), pantothenate (17*µ*M; Sigma), biotin (1*µ*M; Sigma), and EGF (0.8 nM; Hoffmann-La Roche, Basel, Switzerland). Differentiation was induced by adding dexamethasone (DEX; 1*µ*M; Sigma), 3-methyl-isobutyl-1-methylxanthine (MIX; 0.5 mM; Sigma), and bovine insulin (5 *µ*g/ml; Invitrogen). Four days after confluence (d4), cells were kept in SF medium, containing bovine insulin (5 µg/ml) until differentiation was completed. At various time points of the differentiation procedure, beginning with day 0 (day of confluence) and day 2 (hormone addition; for example, dexamethasone and 3-isobutyl-1-methylxanthine), up to 10 days of differentiation, suitable aliquots of cells were taken every two days.

At various time points of the differentiation procedure, beginning with day 0 (day of confluence and hormone addition, for example, insulin), up to 10 days of differentiation, suitable aliquots of cells were taken every two days.

### Expression Profile Analysis of the proteins of the invention

RNA was isolated from mouse tissues or cell culture cells using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art. Total RNA was reverse transcribed (preferably using Superscript II RNaseH- Reverse Transcriptase, from Invitrogen, Karlsruhe, Germany) and subjected to Taqman analysis preferably using the Taqman 2xPCR Master Mix (from Applied Biosystems, Weiterstadt, Germany; the Mix contains according to the Manufacturer for example AmpIiTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

For the analysis of the expression of PTPRB, taqman analysis was performed using the following primer/probe pairs:
Mouse PTPRB forward primer (Seq ID NO: 10) 5'-GAG AAT ACA TCG CCA CTC AGG G-3';
Mouse PTPRB reverse primer (Seq ID NO: 11) 5'-CTC CCA CGC CAT CTT CCA-3';
Mouse PTPRB Taqman probe (Seq ID NO: 12) (5/6-FAM) CGC TTC CAG GCA CCA AGG ATG ACT T (5/6-TAMRA)

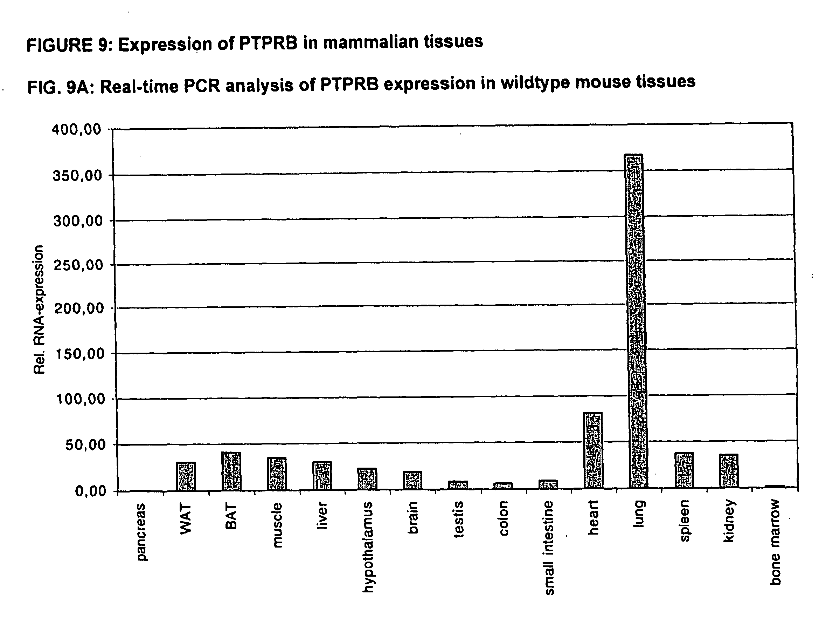

Expression profiling studies confirm the particular relevance of PTPRB as regulator of enery metabolism in mammals. PTPRB is rather ubiquitously expressed, with highest expression levels in lung tissue (see FIGURE 9A).

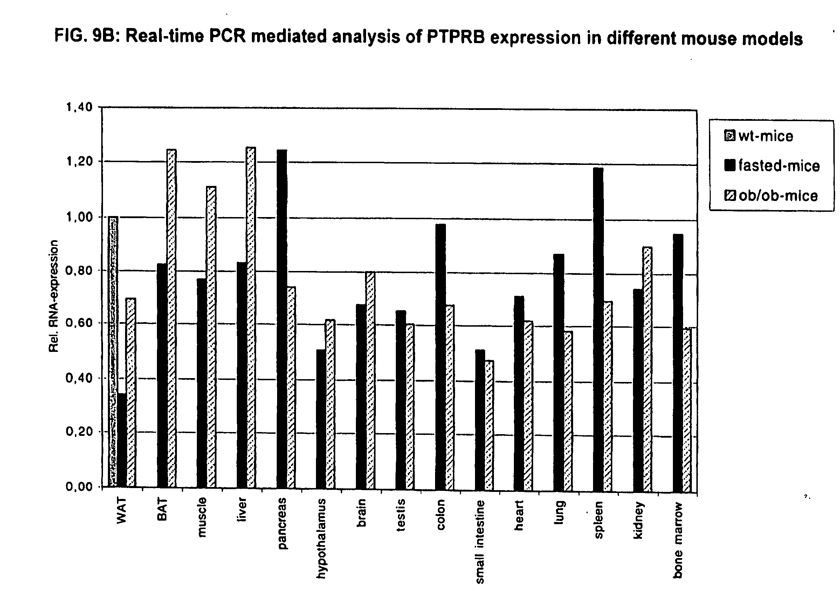

Further, we show that the mammalian homologue of the Drosophilia PTPRB gene, is regulated by fasting. In this invention, we used mouse models of insulin resistance and/or diabetes, such as mice carrying gene knockouts in the leptin pathway (for example, ob (leptin) or db (leptin receptor) mice) to study the expression of the protein of the invention. Such mice develop typical symptoms of diabetes , show hepatic lipid accumulation and frequently have increased plasma lipid levels (see Bruning et al, 1998, Mol. Cell. 2:449-569). We found, for example, that the expression of PTPRB is strongly downregulated in the metabolically active tissue (for example, WAT) of fasted mice (see FIGURE 9B).

## Claims

1. Use of a nucleic acid molecule of protein tyrosine phosphatase PTP10D gene or use of a polypeptide encoded thereby or use of an antibody, an aptamer recognizing said nucleic acid molecule or said polypeptide encoded thereby for the manufacture of a medicament for the treatment, alleviation and/or prevention of obesity and related disorders, selected from obesity, body weight regulation disorders, eating disorder, cachexia, diabetes mellitus, hypertension, coronary heart disease, hypercholesterolemia or dyslipidemia,
wherein
(i) the nucleic acid molecule is a nucleic acid encoding a Drosophila PTP10D (GadFly Accession Number CG1817), human PTP10D homologous protein (PTPRB, Genbank Accession Number XM_006789.7 for the cDNA (SEQ ID NO:3), Genbank Accession Number XP_006789.4 for the protein (SEQ ID NO:4)), and mouse receptor-type protein tyrosine phosphatase (Genbank Accession Number AF157628 for the cDNA, Genbank Accession Number AAF80346 for the protein), or
(ii) wherein said nucleic acid molecule
(a) hybridizes at 50°C in a solution containing 1 x SSC and 0.1% SDS to a nucleic acid molecule as defined in (i) or a nucleic acid molecule which is complementary thereto;
(b) is degenerate with respect to the nucleic acid molecule of (a),
(c) encodes a polypeptide which is at least 85%, preferably at least 90%, more preferably at least 95%, more preferably at least 98% and up to 99,6% identical to PTP10D polypeptides as defined in (i);
(d) differs from the nucleic acid molecule of (a) to (c) by mutation and wherein said mutation causes an alteration, deletion, duplication or premature stop in the encoded polypeptide.

2. The use of claim 1, wherein the medicament further comprises pharmaceutically acceptable carriers, diluents and/or adjuvants.

3. The use of claim 1 or 2, wherein the nucleic acid molecule is a DNA molecule, particularly a cDNA or a genomic DNA.

4. The use of any one of claims 1-3, wherein said nucleic acid encodes a polypeptide contributing to regulating the energy homeostasis and/or the metabolism of triglycerides.

5. The use of any one of claims 1-4, wherein said nucleic acid molecule is a recombinant nucleic acid molecule.

6. The use of any one of claims 1-5, wherein the nucleic acid molecule is a vector, particularly an expression vector.

7. The use of any one of claims 1-4, wherein the polypeptide is a recombinant polypeptide.

8. The use of claim 7, wherein said recombinant polypeptide is a fusion polypeptide.

9. The use of any one of claims 1-6, wherein said nucleic acid molecule is selected from anti-sense oligonucleotides.

## Patentansprüche

1. Verwendung eines Nukleinsäuremoleküls des Gens der Proteintyrosinphosphatase PTP10D oder Verwendung eines **dadurch** kodierten Polypeptids oder Verwendung eines Antikörpers, wobei ein Aptamer das Nukleinsäuremolekül oder das **dadurch** kodierte Polypeptid erkennt, zur Herstellung eines Medikaments zur Behandlung, Linderung und/oder Vorbeugung von Fettsucht und verwandten Störungen ausgewählt aus Fettsucht, Störungen der Körpergewichtsregulierung, Essstörung, Abmagerung, Diabetes mellitus, Bluthochdruck, koronarer Herzerkrankung, Hypercholesterinämie oder Dyslipidämie,
wobei
(i) das Nukleinsäuremolekül eine Nukleinsäure ist, die Drosophila PTP10D (GadFly-Zugangsnummer CG1817), Human-PTP10D-homologes Protein (PTPRB, Genbank-Zugangsnummer XM_006789.7 für die cDNA (SEQ ID NR:3), Genbank-Zugangsnummer XP_006789.4 für das Protein (SEQ ID NR:4)), und Proteintyrosinphosphatase vom Mäuserezeptor-Typ (Genbank-Zugangsnummer AF157628 für die cDNA, Genbank-Zugangsnummer AAF80346 für das Protein) kodiert, oder
(ii) wobei das Nukleinsäuremolekül
(a) bei 50 °C in einer Lösung, enthaltend 1 x SSC und 0,1 % SDS, zu einem wie in (i) definierten Nukleinsäuremolekül oder einem dazu komplementären Nukleinsäuremolekül hybridisiert;
(b) in Bezug auf das Nukleinsäuremolekül von (a) degeneriert ist,
(c) ein Polypeptid kodiert, das zu mindestens 85 %, vorzugsweise zu mindestens 90 %, stärker bevorzugt zu mindestens 95 %, stärker bevorzugt zu mindestens 98 % und bis zu 99,6 % mit wie in (i) definierten PTP10D-Polypeptiden identisch ist;
(d) sich von dem Nukleinsäuremolekül von (a) bis (c) durch eine Mutation unterscheidet, und wobei die Mutation eine Abänderung, Deletion, Verdopplung oder einen vorzeitigen Stopp im kodierten Polypeptid bewirkt.

2. Verwendung nach Anspruch 1, wobei das Medikament ferner pharmazeutisch verträgliche Träger, Verdünnungsmittel und/oder Hilfsstoffe umfasst.

3. Verwendung nach Anspruch 1 oder 2, wobei das Nukleinsäuremolekül ein DNA-Molekül, insbesondere eine cDNA oder eine genomische DNA ist.

4. Verwendung nach einem der Ansprüche 1-3, wobei die Nukleinsäure ein Polypeptid kodiert, das zur Regulierung der Energiehomöostase und/oder des Stoffwechsels von Triglyceriden beiträgt.

5. Verwendung nach einem der Ansprüche 1-4, wobei das Nukleinsäuremolekül ein rekombinantes Nukleinsäuremolekül ist.

6. Verwendung nach einem der Ansprüche 1-5, wobei das Nukleinsäuremolekül ein Vektor, insbesondere ein Expressionsvektor ist.

7. Verwendung nach einem der Ansprüche 1-4, wobei das Polypeptid ein rekombinantes Polypeptid ist.

8. Verwendung nach Anspruch 7, wobei das rekombinante Polypeptid ein Kondensationspolypeptid ist.

9. Verwendung nach einem der Ansprüche 1-6, wobei das Nukleinsäuremolekül ausgewählt ist aus Anti-Sense-Oligonukleotiden.

## Revendications

1. Utilisation d'une molécule d'acide nucléique du gène PTP10D de protéine tyrosine phosphatase ou utilisation d'un polypeptide encodé par celle-ci ou utilisation d'un anticorps, d'un aptamère reconnaissant ladite molécule d'acide nucléique ou ledit polypeptide encodé par celle-ci pour la fabrication d'un médicament pour le traitement, l'atténuation et/ou la prévention de l'obésité et de troubles associés, choisis parmi l'obésité, les troubles de régulation du poids corporel, les troubles de l'alimentation, la cachéxie, le diabète sucré, l'hypertension, la maladie coronarienne, l'hypercholestérolémie ou la dyslipidémie,
dans laquelle :
(i) la molécule d'acide nucléique est un acide nucléique encodant un PTP10D de Drosophile (numéro d'accès GadFly CG1817), une protéine homologue PTP10D humaine (PTBRB, numéro d'accès Genbank XM_006789.7 pour l'ADNc (SEQ ID n° 3), numéro d'accès Genbank XP_006789.4 pour la protéine (SEQ ID n° 4)), et une protéine tyrosine phosphatase de souris de type récepteur (numéro d'accès Genbank AF157628 pour l'ADNc, numéro d'accès Genbank AAF80346 pour la protéine), ou
(ii) dans laquelle la molécule d'acide nucléique
(a) s'hybride à 50 °C dans une solution contenant 1 x SSC et 0,1 % de SDS à une molécule d'acide nucléique telle que définie dans (i) ou une molécule d'acide nucléique qui est complémentaire à celle-ci ;
(b) dégénère par rapport à la molécule d'acide nucléique de (a),
(c) encode un polypeptide qui est au moins 85 %, de préférence au moins 90 %, de manière préférée au moins 95 %, de manière préférée au moins 98 % et jusqu'à 99,6 % identique aux polypeptides PTP10D tels que définis dans (i) ;
(d) diffère de la molécule d'acide nucléique de (a) à (c) par mutation et dans laquelle ladite mutation provoque une altération, une délétion, une duplication ou un arrêt prématuré dans le polypeptide encodé.

2. Utilisation selon la revendication 1, dans laquelle le médicament comprend en outre des excipients, diluants et/ou adjuvants pharmaceutiquement acceptables.

3. Utilisation selon la revendication 1 ou 2, dans laquelle la molécule d'acide nucléique est une molécule d'ADN, en particulier un ADNc ou un ADN génomique.

4. Utilisation selon l'une quelconque des revendications 1 à 3, dans laquelle ledit acide nucléique encode un polypeptide contribuant à réguler l'homéostase énergétique et/ou le métabolisme des triglycérides.

5. Utilisation selon l'une quelconque des revendications 1 à 4, dans laquelle ladite molécule d'acide nucléique est une molécule d'acide nucléique recombinante.

6. Utilisation selon l'une quelconque des revendications 1 à 5, dans laquelle la molécule d'acide nucléique est un vecteur, en particulier un vecteur d'expression.

7. Utilisation selon l'une quelconque des revendications 1 à 4, dans laquelle le polypeptide est un polypeptide recombinant.

8. Utilisation selon la revendication 7, dans laquelle ledit polypeptide recombinant est un polypeptide de fusion.

9. Utilisation selon l'une quelconque des revendications 1 à 6, dans laquelle ladite molécule d'acide nucléique est choisie parmi les oligo-nucléotides antisens.
